(19) Europäisches Patentamt
European Patent Office
Office européen des brevets

(11) **EP 2 674 105 B1**

(12) **EUROPÄISCHE PATENTSCHRIFT**

(45) Veröffentlichungstag und Bekanntmachung des Hinweises auf die Patenterteilung:
**28.09.2016 Patentblatt 2016/39**

(51) Int Cl.:
*A61B 5/22* (2006.01)   *G01L 5/16* (2006.01)
*A61C 19/045* (2006.01)   *G01L 5/00* (2006.01)

(21) Anmeldenummer: **12004504.2**

(22) Anmeldetag: **14.06.2012**

(54) **Intraoral verwendbare Sensorvorrichtung**

Dispositif de détection utilisable en intra-oral

Intraoral sensor device

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.12.2013 Patentblatt 2013/51**

(73) Patentinhaber: **Arnold, Linda Maria**
**58452 Witten (DE)**

(72) Erfinder: **Arnold, Linda Maria**
**58452 Witten (DE)**

(74) Vertreter: **Leinweber & Zimmermann**
**Rosental 7, II. Aufgang**
**80331 München (DE)**

(56) Entgegenhaltungen:
WO-A1-92/03106       WO-A2-2008/033852
CN-U- 202 270 028    DE-A1-102005 006 323
DE-A1-102008 006 595

• LI XIAOTAO ET AL: "Research on a Novel Sensor for Measuring Force in Arbitrary Direction", COMPUTER AND ELECTRICAL ENGINEERING, 2008. ICCEE 2008. INTERNATIONAL CONFERENCE ON, IEEE, PISCATAWAY, NJ, USA, 20 December 2008 (2008-12-20), pages 241-244, XP031403360, ISBN: 978-0-7695-3504-3

**Beschreibung**

Die Erfindung betrifft eine Sensorvorrichtung zur intraoralen Messung einer Kieferstellung, einer Kieferbewegung und/oder einer Kieferkraft mit einem Gehäuse, einem in dem Gehäuse aufgenommenen Verformungskörper, einer mit dem Verformungskörper verbundenen Druckplatte zur Übertragung einer lokal auf die Druckplatte einwirkenden externen Kraft auf den Verformungskörper, so dass sich der Verformungskörper zumindest lokal verformt, und einem an dem Verformungskörper angeordneten Sensor zur Messung einer Verformung des Verformungskörpers. Außerdem betrifft die Erfindung einen Verformungskörper für eine solche Sensorvorrichtung.

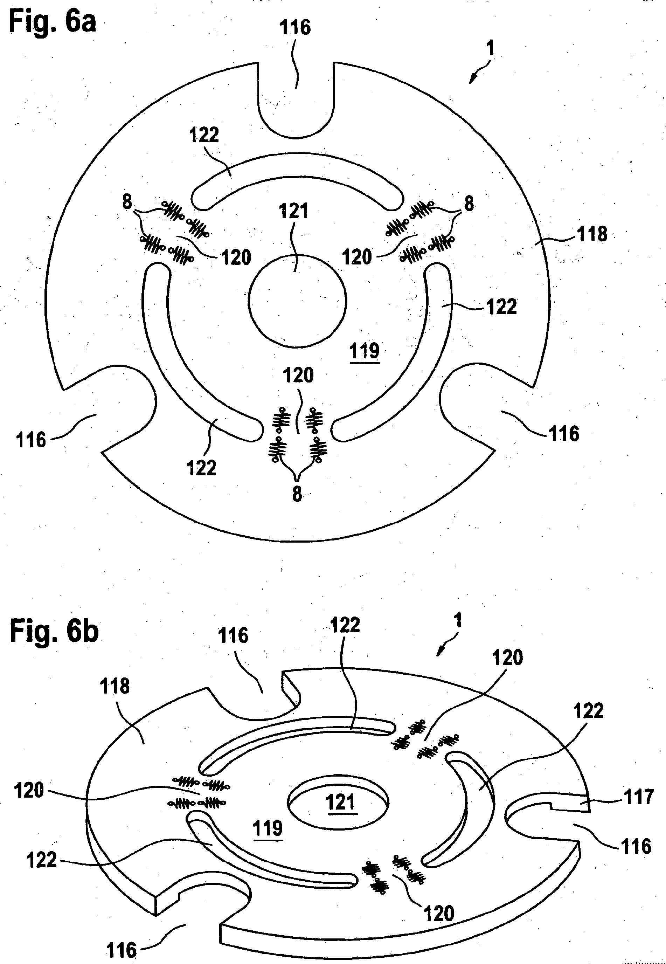

Intraoral einsetzbare Sensorvorrichtungen sind bereits bekannt. So ist in der DE 10 2005 006 323 A1 eine Sensorvorrichtung beschrieben, mit der ein Bewegungsverlauf und eine Kraft eines beweglichen Körpers oder Körperteils gegenüber einem festen Gehäuse ermittelt werden können. In Fig. 6 der genannten Schrift ist eine herkömmliche Sensorvorrichtung in Verwendung gezeigt: Ein Bewegungs- und Kraftinitiator ist am Oberkiefer eines Probanden angeordnet und eine Sensorvorrichtung ist im Bereich des Unterkiefers des Probanden abgesetzt. Wenn nun der Proband Ober- und Unterkiefer einander annähert, kommt ein Spitzenende des Kraftinitiators in Kontakt mit einer Krafteinleitungsfläche der Sensorvorrichtung, und die auf die Krafteinleitungsfläche einwirkende Kraft wird von der Sensorvorrichtung ermittelt. Wenn der Proband nun Ober- und Unterkiefer relativ zueinander bewegt, bewegt sich das Spitzenende entlang einer Bewegungsbahn auf der Krafteinleitungsfläche, wobei diese Bewegungsbahn ermittelt und dann angezeigt und/oder digital ausgewertet und abgespeichert werden kann.

Die in der DE 10 2005 006 323 A1 beschriebene Sensorvorrichtung weist insgesamt drei Messsensoren auf. Wenn sich bei dieser Vorrichtung der Kraftinitiator beispielsweise über dem Mittelpunkt der Krafteinleitungsfläche befindet, detektiert die Vorrichtung die gleiche Amplitude der elektrischen Signale aller drei Sensoren. Bei einer Bewegung des Kraftinitiators weg von dem Mittelpunkt der Krafteinleitungsfläche ändern sich die Amplituden der Messsignale der drei Sensoren relativ zueinander, woraus die Bewegungsbahn des Kraftinitiators auf der Krafteinleitungsfläche bestimmbar ist. Der Betrag der Kraft, mit der der Kraftinitiator auf die Krafteinleitungsfläche drückt, kann über eine vektorielle Addition der Einzelsignale berechnet werden. Insgesamt drei geeignet angeordnete Sensoren und damit drei Messwerte genügen dabei, um zu jedem Zeitpunkt den Betrag der auf die Krafteinleitungsfläche wirkenden Kraft (die "Kiefer(schluss)kraft") und die Position des Kraftinitiators auf der Krafteinleitungsfläche (den Krafteinleitungsort und damit die "Kieferstellung") bestimmen zu können.

Auf diese Weise können bestimmte Bewegungsabläufe zwischen Ober- und Unterkiefer erfasst, angezeigt und abgespeichert werden und bestimmte Kieferstellungen können reproduzierbar vermessen und dann analysiert werden. Im Hinblick auf dieses als Kieferrelationsbestimmung (KRB) bezeichnete Messverfahren wird beispielhaft auf die Veröffentlichung "Zur Lagebestimmung des Unterkiefers in zentrischer Relation mit dem DIR-System" von A. Dietzel und A. Zöllner (Publikation: Deutscher Ärzte Verlag, DZZ, Deutsche Zahnärztliche Zeitschrift, 2012; 67 (2)) verwiesen, deren Inhalt durch Verweis in die vorliegende Offenbarung mit aufgenommen wird.

Bei der in der DE 10 2005 006 323 A1 beschriebenen Sensorvorrichtung sind die Sensoren jeweils auf einem Grundkörper aus elastischem Kunststoff angeordnet, der sich bei Einwirkung einer externen Kraft auf die Krafteinleitungsfläche elastisch verformt. Die externe Kraft wird dabei über eine zwischen Grundkörper und Krafteinleitungsfläche angeordnete weitere elastische Kunststoffschicht auf den elastischen Grundkörper übertragen.

Es hat sich gezeigt, dass die mit dieser Sensorvorrichtung ermittelten Positions- und Kraftwerte nicht immer zuverlässig reproduzierbar sind. Ferner ist derjenige Bereich der Krafteinleitungsfläche, in dem eine Bestimmung des Krafteinleitungsortes exakt möglich ist, im Wesentlichen begrenzt auf den oberhalb der Sensoren liegenden Bereich der Krafteinleitungsfläche, der bei der Sensorvorrichtung der DE 10 2005 006 323 A1 etwa dreiecksförmig ist. Außerhalb dieses Dreiecks" nimmt die Messgenauigkeit stark ab.

Die Druckschrift WO 92/03106 offenbart eine Vorrichtung zum Messen einer Kiekskraft mit einem plattenförmigen Verformungskörper.

In Anbetracht dieser Probleme ist es die Aufgabe der vorliegenden Erfindung, eine Sensorvorrichtung zur intraoralen Messung einer Kieferstellung, einer Kieferbewegung und/oder einer Kieferkraft bereitzustellen, die zuverlässig und unabhängig vom Ort der Krafteinwirkung reproduzierbare Messwerte liefert.

Diese Aufgabe wird durch eine Weiterbildung der bekannten Sensorvorrichtung gemäß Anspruch 1 gelöst. Mit anderen Worten ist der einen Randabschnitt bildende Stützabschnitt gegenüber dem Gehäuse feststehend, und der zentrale Krafteinleitungsabschnitt ist bei Krafteinleitung gegenüber dem Stützabschnitt bewegbar, insbesondere verkippbar und/oder in Krafteinleitungsrichtung versetzbar, wobei dieser Versatz bzw. diese Verkippung zu einer Verformung im Biegeabschnitt führen, die dann durch den Sensor messbar ist.

Diese Verschwenkung bzw. dieser Versatz zwischen Stützabschnitt und Krafteinleitungsabschnitt bei Krafteinwirkung sind bevorzugt nur äußerst gering, so dass sie sich im Biegeabschnitt in einer geringen, aber messbaren Stauchung bzw. Streckung der Oberfläche des Verformungskörpers äußern.

Die Erfindung geht auf die Erkenntnis zurück, dass das Verformverhalten von Körpern aus elastischen Kunststoffen in Abhängigkeit von der Entfernung vom Verformungszentrum sehr komplex ist, und eine elastische Zwischenschicht zwischen Krafteinleitungsfläche und elastischem Körper das Verformverhalten weiter verkompliziert. Lokal im Bereich des Verformungszentrums ist die Verformung nämlich generell stark, jedoch ist der elastische Körper auch in Bereichen, die vom Verformungszentrum beabstandet sind, mehr oder weniger stark verformt, wobei eine allgemeine Verformungskurve nur schwer bestimmbar ist. Zusätzlich sind bei herkömmlichen Sensorvorrichtungen großflächige Sensoren erforderlich, um zu einer genauen Ortsmessung in einem möglichst großen Bereich der Druckplatte kommen zu können. Großflächige Sensoren sind jedoch problematisch, insbesondere wenn sie über ihre gesamte Länge nicht gleichmäßig verformt werden.

Die erfindungsgemäße Sensorvorrichtung weist dagegen einen Verformungskörper mit einem feststehenden Stützabschnitt auf, der über einen Biegeabschnitt mit einem Krafteinleitungsabschnitt gekoppelt ist, so dass der zentrale Krafteinleitungsabschnitt gegenüber dem Stützabschnitt bewegbar ist. Damit wird bei der erfindungsgemäßen Sensorvorrichtung der Verformungskörper generell nur in seinem Biegeabschnitt, in dem der Sensor angeordnet ist, verformt, und zwar unabhängig davon, an welcher Stelle die Kraft in den Krafteinleitungsabschnitt eingeleitet wird. Aus der Stärke dieser Verformung und damit aus dem Sensorsignal können nun der Kraftbetrag und/oder der Ort bestimmt werden, an dem die Kraft auf die Druckplatte einwirkt.

Im Übrigen kann der Biegeabschnitt der erfindungsgemäßen Sensorvorrichtung klein ausgestaltet sein, da sich auch eine vom Biegeabschnitt entfernte Krafteinwirkung in den Krafteinleitungsabschnitt über die Druckplatte in einer lokalen Verformung des Biegeabschnitts zeigt, so dass keine großflächigen Sensoren wie bei den herkömmlichen Vorrichtungen erforderlich sind.

Der Biegeabschnitt, in dem der Verformungskörper bei einer Krafteinwirkung verformt wird, ist vorzugsweise klein gegenüber dem Krafteinleitungsabschnitt und dem Stützabschnitt. Sein Volumen kann weniger als 20%, bevorzugt weniger als 10%, insbesondere 5% oder weniger des Gesamtvolumens des Verformungskörpers ausmachen. So wird genau festgelegt, an welcher Stelle des Verformungskörpers die Verformung auftritt, was die Verformung vorhersehbar und exakt messbar macht.

Vorzugsweise ist der Verformungsköper einteilig gebildet. Wenn Stützabschnitt, Biegeabschnitt und Krafteinleitungsabschnitt einteilig mit vorgegebenen Abmessungen aus einem vorgegebenen Material hergestellt werden, ist das Biegeverhalten des Verformungskörpers sehr exakt festlegbar, und es lassen sich mit verschiedenen erfindungsgemäßen Sensorvorrichtungen, die mit gleichen Verformungskörpern ausgestattet sind, besonders reproduzierbare Messwerte erzielen.

Der Verformungskörper ist vorzugsweise flach, insbesondere etwa plattenförmig. Ein plattenförmiger Verformungskörper ist besonders einfach herstellbar. Außerdem ist eine Platte einfach in der Handhabung und praktisch für den Einbau in ein Gehäuse. Eine Verschwenkung und/oder ein Versatz zwischen zwei Abschnitten einer Platte sind besonders gut messbar. Durch Modifikation von Plattenstärke oder Plattenmaterial kann auf den Messbereich und die Messgenauigkeit eingewirkt werden.

Besonders bevorzugt ist der Verformungskörper eine einteilige Metallplatte wie etwa eine Aluminium- oder Stahlplatte, insbesondere eine Edelstahlplatte. Der Elastizitätsmodul dieser Materialien liegt in einem geeigneten Bereich und verändert sich zeitlich auch nach zahlreichen Verformungen bei wiederholter Krafteinwirkung kaum. Im Übrigen ist das Elastizitätsverhalten dieser Materialien annähernd linear. Auch andere Materialien sind denkbar, insbesondere Materialien, deren Elastizitätsverhalten noch weniger temperaturabhängig ist.

Der Biegeabschnitt kann dadurch gebildet sein, dass der Verformungskörper in seiner Umgebung mindestens eine lokale Schwächung aufweist. In der Umgebung einer lokalen Schwächung verformt sich ein Körper bei Belastung nämlich stärker als in einem ungeschwächten Bereich. Durch Versehen des Verformungskörpers mit einer lokalen Schwächung kann ein wirksamer Biegeabschnitt erzeugt werden, der einfach in der Herstellung ist. Bspw. ist der Verformungskörper eine Platte mit zwei oder mehr Durchbrüchen, zwischen denen der Biegeabschnitt angeordnet ist.

Die lokale Schwächung kann in Form eines oder mehrerer Durchbrüche durch den Verformungskörper, einer Materialabtragung an dem Verformungskörper und/oder einer lokalen Verringerung der Stärke des Verformungskörpers gebildet ist. Wenn der Verformungskörper einteilig gebildet und plattenförmig ist, können diese Durchbrüche und/oder Materialabtragungen bei der Herstellung des Verformungskörpers und vor dessen Einsetzen in das Gehäuse aus einer zunächst ungeschwächten und lochfreien Platte herausgefräst oder herausgeschnitten werden.

Bei einer besonders bevorzugten Ausführungsform ist der Biegeabschnitt in Form eines den Krafteinleitungsabschnitt mit dem Stützabsehnitt verbindenden Stegs gebildet. Unter Steg wird in der vorliegenden Offenbarung eine zumindest abschnittsweise schmale Materialbrücke verstanden, die entlang einer Längsrichtung verläuft, und an deren Seite jedenfalls abschnittsweise mindestens ein Durchbruch durch den Verformungskörper angeordnet ist. Die Materialstärke des Verformungskörpers im Bereich des Stegs in der Krafteinleitungsrichtung kann abschnittsweise kleiner sein als die Materialstärke des übrigen Verformungskörpers. Durch eine Anpassung Stegdicke kann das Verformungsverhalten des Verformungskörpers im Biegeabschnitt gesteuert werden. Auch kann die Materialstärke eines ersten Stegabschnitts größer sein als die Materialstärke eines zweiten Stegabschnitts. Auf diese Weise kann gesteuert werden, an welchen Biegestellen des als Steg ausgeführten Biegeabschnitts sich der Verformungskörper bei einer Krafteinwirkung auf den Krafteinleitungsbereich besonders stark verformt. Der Sensor kann dann an den entsprechenden Bereichen angeordnet werden.

Der Krafteinleitungsabschnitt ist ein zentraler Abschnitt des Verformungskörpers und der Steg kann in radialer Richtung nach außen in Richtung auf den Stützabschnitt verlaufen, der auf einem Absatz des Gehäuses aufliegt und den Krafteinleitungsabschnitt umläuft.

Alternativ kann der Steg kann ein Doppelsteg sein, dessen beide Enden mit dem Krafteinleitungsabschnitt verbunden sind und dessen Mitte mit dem Stützabschnitt verbunden ist oder umgekehrt, so dass der Biegeabschnitt eine erste Biegestelle zwischen der Stegmitte und dem ersten Stegende und eine zweite Biegestelle zwischen der Stegmitte und dem zweiten Stegende hat. Eine weitere Biegestelle kann alternativ oder zusätzlich in einem Bereich angeordnet sein, der von dem Rand des zentralen Abschnitts des Verformungskörpers in Richtung auf die Mitte des Doppelstegs verläuft. Die dritte Biegestelle verläuft in radialer Richtung.

Ein Biegeabschnitt in Form eines Doppelstegs hat sich im Rahmen einer guten Messgenauigkeit als besonders geeignet erwiesen. Dies liegt daran, dass bei geeigneter Anordnung des Doppelstegs an den einzelnen Biegestellen des Doppelstegs verschiedene Kräfte gemessen werden können, so dass insgesamt nicht nur der Kraftbetrag und der Krafteinleitungsort messbar sind, sondern auch die Krafteinleitungsrichtung und/oder Torsions- und Querkräfte. Alternativ oder zusätzlich kann der Doppelsteg so angeordnet sein, dass eine erste Biegestelle des Stegs gestaucht wird, wenn eine zweite Biegestelle in demselben Maß gestreckt wird und umgekehrt. Diese Stauch- und Streckabschnitte können für eine Anbringung einzelner Messwiderstände eines Sensors verwendet werden, um Störgrößen zu kompensieren.

Der Sensor zur Messung der Verformung des Verformungskörpers ist vorzugsweise an dem und/oder angrenzend an den Steg angeordnet, da hier die Verformung des Verformungskörpers besonders stark und damit genau messbar ist. Der Sensor kann in der Mitte und/oder im Sockelbereich des Stegs angeordnet sein. Ferner können Sensoren bzw. können verschiedene Messwiderstände eines Sensors sowohl an einem Streckabschnitt als auch an einem Stauchabschnitt eines Stegs angeordnet sein. Dabei können verschiedene Messwiderstände eines Sensors sowohl an der Oberseite des Stegs als auch an der Unterseite des Stegs angeordnet sein. Wenn nämlich die Oberfläche der Oberseite eines Stegs gestreckt wird, wird die Oberfläche der Unterseite gestaucht und umgekehrt. Der Steg kann zu diesem Zweck in der Krafteinleitungsrichtung besonders dünn ausgeführt sein, so dass Stauchung und Streckung einander (mit umgekehrtem Vorzeichen) besonders gut entsprechen.

Alternativ kann ein erster Messwiderstand eines Sensors an einer Oberfläche eines radial inneren Bereichs eines radial nach außen verlaufenden Stegs angeordnet sein und ein zweiter Messwiderstand des Sensors kann an der Oberfläche eines radial äußeren Bereichs des Stegs angeordnet sein. Wenn nämlich das freie Ende eines einseitig befestigten Stegs durch Belastung parallel nach unten versetzt wird, verformt sich der Steg und seine Oberfläche bildet in einer entlang der Längsrichtung des Stegs verlaufenden Schnittebene eine S-Form aus. Mit anderen Worten wird die Oberfläche des inneren Stegbereichs bei Belastung gestreckt, wenn die Oberfläche des äußeren Stegbereichs gestaucht wird und umgekehrt. Auch diese Stauch- und Streckabschnitte können für eine Anbringung einzelner Messwiderstände eines Sensors verwendet werden, um Störgrößen zu kompensieren.

Sowohl der Ort der Krafteinleitung und damit die Kieferstellung als auch der Betrag der einwirkenden Kraft und damit die Kieferkraft sind ermittelbar, weil der Verformungskörper mindestens zwei, bevorzugt drei Biegeabschnitte aufweist, an denen jeweils ein Sensor zur Messung einer Verformung des Verformungskörpers angeordnet ist. Aus den drei Messwerten können die gesuchten Größen in erster Näherung mithilfe eines linearen Gleichungssystems bzw. über eine Matrizengleichung ermittelt werden. Der Ort der Krafteinleitung kann bspw. in Polarkoordinaten oder in kartesischen Koordinaten gegenüber einem Zentrum des Verformungskörpers bestimmt und über eine Anzeigevorrichtung angezeigt bzw. in einer Datenverarbeitungseinrichtung gespeichert werden. Eine Kieferbewegung wird ermittelt, indem der Ort der Krafteinleitung kontinuierlich oder in bestimmten Zeitabständen ermittelt und die in bestimmten Zeitabständen durchlaufenen Krafteinleitungsorte angezeigt und/oder gespeichert werden.

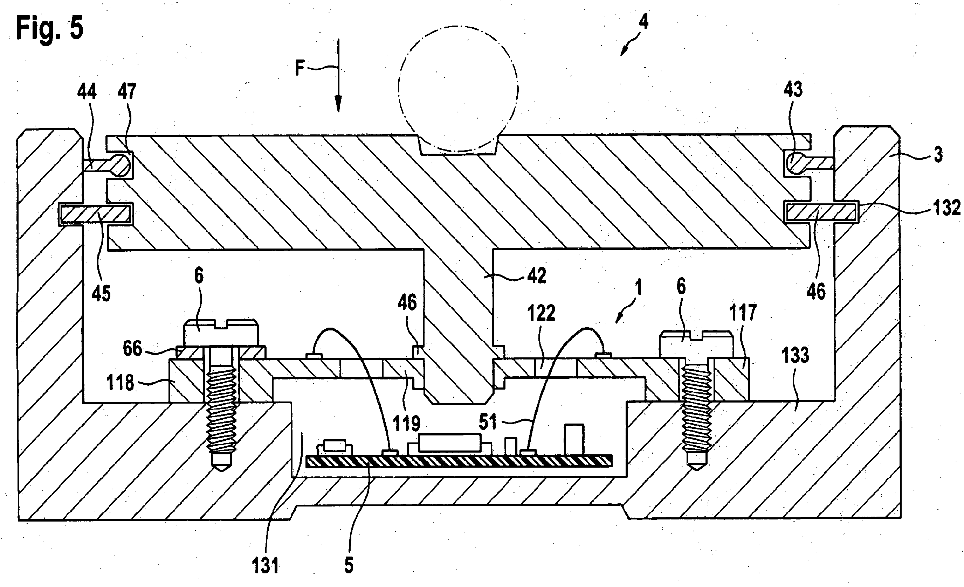

Der Krafteinleitungsabschnitt ist ein zentraler Abschnitt des Verformungskörpers und der Stützabschnitt ein den zentralen Abschnitt umlaufender Randabschnitt, wobei der zentrale Abschnitt über die Biegeabschnitte mit dem Randabschnitt verbunden ist. Der Stützabschnitt kann den Krafteinleitungsabschnitt teilweise, besonders bevorzugt vollständig umlaufen. Er kann über eine oder mehrere Fixierstellen fest mit dem Gehäuse verbunden sein. Besonders bevorzugt ist er etwa ringförmig, liegt auf einem etwa ringförmigen Auflageabsatz des Gehäuses auf und ist an diesem Auflageabsatz befestigt. Die Biegeabschnitte können in Umfangsrichtung verteilte Stege sein, die jeweils den Stützabschnitt mit dem zentralen Krafteinleitungsabschnitt verbinden. Die Kraft kann über ein mit der Druckplatte verbundenes oder einteilig damit gebildetes Krafteinleitungselement in einen zentralen Bereich des Krafteinleitungsabschnitts eingeleitet werden. Eine solche Ausführungsform der Erfindung ist in den Figuren 5 bis 8 gezeigt.

Mit einer solchen Ausgestaltung des Verformungskörpers sind genauere und reproduzierbarere Messungen der Kieferstellung möglich als mit den aus dem Stand der Technik bekannten Sensorvorrichtungen, bei denen die drei Sensoren auf drei, in verschiedenen Kammern befindlichen elastischen Grundkörpern angeordnet sind. Es sind nämlich bei erfindungsgemäßen Sensorvorrichtungen mit einteilig gebildetem Verformungskörper mit mehreren Biegeabschnitten im unverformten Zustand des Verformungskörpers sowohl die relative Lage von Stützabschnitt und Krafteinleitungsabschnitt als auch die relativen Lagen der einzelnen Biegeabschnitte zueinander jeweils gleich, während sich bei herkömmlichen Vorrichtungen die relativen Lagen der einzelnen Kammern zueinander herstellungsbedingt unterscheiden können. Dies kann bei Verwendung einer baugleichen anderen herkömmlichen Sensorvorrichtung bei identischer Krafteinwirkung zu unterschiedlichen Messwerten führen.

Bei einer erfindungsgemäß bevorzugten Ausführungsform sind die Biegeabschnitte jeweils als sich quer, insbesondere etwa senkrecht zur Radialrichtung erstreckende Doppelstege ausgeführt, die jeweils an einem zentralen Abschnitt davon mit dem Stützabschnitt und an den beiden Endabschnitten davon mit dem Krafteinleitungsabschnitt verbunden sind oder umgekehrt. Die Doppelstege sind vorzugsweise im Wesentlichen achsensymmetrisch bzgl. einer radial nach außen durch ihre Mitte verlaufenden Symmetrieachse. Der Winkel zwischen zwei benachbarten Doppelstegen kann jeweils 120° betragen, so dass insgesamt drei in Umfangsrichtung verteilte Doppelstege vorhanden sind. An jedem Doppelsteg kann ein Sensor zur Messung einer Verformung des jeweiligen Doppelstegs angeordnet sein. Diese symmetrische Anordnung macht eine Ermittlung der zu bestimmenden Größen aus den Sensorenmesswerten einfacher.

Besonders bevorzugt hat der Verformungskörper eine 3-zählige Radiärsymmetrie Der Verformungskörper kann drei gleich geformte Biegeabschnitte aufweisen, die gegenseitig jeweils einen Winkel von 120° einschließen. Zusätzlich oder alternativ kann der Krafteinleitungsabschnitt drei Befestigungsstellen aufweisen, über die die Druckplatte an dem Krafteinleitungsabschnitt befestigt ist. Diese Befestigungsstellen können gegenseitig jeweils einen Winkel von 120° einschließen und jeweils bevorzugt mittig zwischen zwei Biegeabschnitten angeordnet sein. Dadurch ist eine Proportionalität zwischen den gemessenen Signalen und den an den drei Befestigungsstellen in den Krafteinleitungsabschnitt eingeleiteten Teillasten gegeben.

Zusätzlich oder alternativ kann der Verformungskörper einen kreisförmigen Umriss haben. Zusätzlich oder alternativ kann der Verformungskörper eine oder mehrere Gruppen von Durchbrüchen aufweisen, wobei jede Gruppe aus bevorzugt drei in Umfangsrichtung verlaufenden Durchbrüchen besteht. Die benachbarten Durchbrüche einer Gruppe können jeweils gegenseitig einen Winkel von 120° einschlie ßen. Zwischen den Durchbrüchen können die Biegeabschnitte gebildet sein. Die Durchbrüche können als die Mitte des Stützabschnitts bogenförmig umlaufende Schlitze ausgebildet sein, zwischen denen jeweils ein radial nach außen verlaufender Steg angeordnet ist, der jeweils mindestens einen Sensor trägt. Zusätzlich oder alternativ kann eine Gruppe von Durchbrüchen als senkrecht zur Radialrichtung verlaufende Schlitze bereitgestellt sein, die in Radialrichtung außerhalb der bogenförmig verlaufenen Schlitze angeordnet sind und mit ihnen in Umfangsrichtung teilweise überlappen. Auf diese Weise können insgesamt drei in Umfangsrichtung angeordnete Doppelstege bereitgestellt werden. Die 3-zählige Radiärsymmetrie vereinfacht die Auswertung der Messwerte der Sensoren. Außerdem wird die Fehleranfälligkeit der Sensorvorrichtung bei der Herstellung vermindert.

Als besonders zweckmäßig hat es sich herausgestellt, die Sensoren jeweils als Messwiderstande, bevorzugt als Dehnungsmessstreifen (DMS) auszubilden. Die Messwiderstände sind mit einer Verstärkerschaltung und/oder einer Auswertelektronik verbunden, die im Inneren des Gehäuses auf einer Platine angeordnet sein kann. Alternativ oder zusätzlich kann eine Auswertelektronik in einer Auswertvorrichtung außerhalb des Gehäuses angeordnet sein.

Vorzugsweise sind die Messwiderstände in Dünnfilmtechnik auf den Verformungskörper aufgetragene Widerstände. Bei der Herstellung hat es sich als zweckmäßig erwiesen, zunächst die Messwiderstände an dem Verförmungskörper anzubringen und anschließend eine oder mehrere lokale Schwächungen in den Verformungskörper in Bereiche einzubringen, die dann die Biegeabschnitte bilden.

Jeder Sensor kann einen, zwei oder vier Messwiderstände aufweisen, die in einer Brückenschaltung (Viertelbrücke, Halbbrücke oder Vollbrücke) verschaltet sind.

In einer besonders bevorzugten Ausführungsform der Erfindung weist jeder Sensor vier zu einer Vollbrücke geschaltete Dehnungsmessstreifen auf Insbesondere sind diese Vollbrücken temperaturkompensierte Wheatstone'sche Vollbrücken. Hierzu können jeweils zwei Messwiderstände eines Sensors in einer druck- bzw. zugbeanspruchten Zone auf der Oberfläche des Sensors angeordnet sein.

Als vorteilhaft hat sich herausgestellt, wenn der Sensor mindestens einen entlang einer ersten Biegefläche des Verformungskörpers angeordneten ersten Dehnungsmessstreifen und einen entlang einer zweiten Biegefläche des Verformungskörpers angeordneten zweiten Dehnungsmessstreifen aufweist, wobei die erste Biegefläche infolge einer Krafteinwirkung auf die Druckplatte gestreckt wird (Streckabschnitt), wenn die zweite Biegefläche gestaucht wird (Stauchabschnitt) und umgekehrt, und wobei der erste Dehnungsmessstreifen und der zweite Dehnungsmessstreifen zu einer Halbbrücke oder zu einer Vollbrücke geschaltet sind. Besonders bevorzugt weist der Sensor insgesamt vier Messwiderstände auf, von denen zwei in einem Stauchabschnitt eines Stegs und die anderen zwei in einem Streckabschnitt des Stegs angeordnet sind.

Durch eine Temperaturkompensation infolge der Schaltung zu einer Vollbrücke werden die Messergebnisse exakter und reproduzierbarer.

Besonders vorteilhaft ist es, wenn der Krafteinleitungsabschnitt fest mit der Druckplatte gekoppelt ist, so dass die relative Lage von Druckplatte und Krafteinleitungsabschnitt bei Krafteinwirkung auf die Druckplatte gleichbleibend ist. Bevorzugt wird die Druckplatte auf Abstand zu dem Krafteinleitungsabschnitt gehalten. Zu diesem Zweck kann mindestens ein Abstandhalteelement zwischen die Druckplatte und den Krafteinleitungsabschnitt eingebracht sein, durch das diese relative Lage festlegbar ist und gleichzeitig eine erforderliche Beweglichkeit des Krafteinleitungsabschnittes gegenüber dem Stützabschnitt ermöglicht wird. Krafteinleitungsabschnitt, Abstandhalteelement(e) und/oder Druckplatte können auch einteilig gebildet sein.

Eine Veränderung der Lage der Druckplatte durch die externe Krafteinwirkung hat unmittelbar eine Veränderung der Lage des Krafteinleitungsabschnittes zur Folge, wobei diese Lageänderung des Krafteinleitungsabschnittes gegenüber dem Stützabschnitt an den Biegeabschnitten gemessen wird. Diese Lageänderungen sind äußerst gering, jedoch aufgrund der damit verbundenen Stauchungen und/oder Streckungen des Biegeabschnitts messbar.

Die erfindungsgemäße Sensorvorrichtung ist intraoral optimal verwendbar, wenn das Gehäuse in einer senkrecht zur Krafteinleitungsrichtung verlaufenden Schnittebene abgerundet, bevorzugt etwa kreisförmig ist, und derart geformt ist, dass es zwischen den Molaren eines Unterkiefers absetzbar ist. Der Durchmesser des Gehäuses bzw. dessen Abmessung in Querrichtung ist vorzugsweise kleiner als 4 cm, bevorzugt kleiner als 3 cm und vorzugsweise größer als 1 cm. Zur Verwendung im intraoralen Bereich sollte die erfindungsgemäße Sensorvorrichtung wasserdicht sein, so dass kein Speichel oder andere Körperflüssigkeiten in das Gehäuseinnere eindringen können. Entsprechend ist ein Zwischenraum zwischen dem Gehäuse und der das Gehäuse verschließenden Druckplatte dicht verschlossen. Die Sensoren können die Messwerte zur Weiterverarbeitung drahtlos oder per Kabel an eine Auswert- und/oder Anzeigevorrichtung außerhalb des Gehäuses übertragen.

Bei einer erfindungsgemäß bevorzugten Ausführungsform ist eine Abmessung einer Krafteinleitungsfläche der Druckplatte größer, bevorzugt mehr als 1,5 mal so groß, insbesondere etwa doppelt so groß oder größer wie/als eine maximale Abmessung des Verformungskörpers in einer senkrecht zur Krafteinleitungsrichtung verlaufenden Richtung. Bei erfindungsgemäßen Sensorvorrichtungen ist nämlich nicht notwendigerweise jeder Bereich der Druckplatte oberhalb des Verformungskörpers angeordnet. Vielmehr kann die Druckplatte von ihren äußeren Abmessungen her größer sein als der Verformungskörper und über den Verformungskörper hinausragen. Somit kann erfindungsgemäß eine Sensorvorrichtung mit großer Messfläche bei gleichzeitig kompakter Ausgestaltung von Verformungskörper und Sensor bereitgestellt werden, wodurch Herstellungskosten eingespart werden. Ein Freiraum im Gehäuseinneren unterhalb der Druckplatte und seitlich des Verformungskörpers kann zur Aufnahme einer Platine mit einer Auswertelektronik o. dgl. dienen.

Es hat sich als zweckmäßig herausgestellt, die Biegeabschnitte so auszugestalten, dass mit der Sensorvorrichtung Kieferkräfte im Bereich zwischen 5 N und 100 N, vorzugsweise zwischen 10 N und 50 N messbar sind. Dies ist nämlich der Bereich der Kieferkraft, in dem Kieferrelationsmessungen zweckmäßigerweise durchgeführt werden.

Es ist weiterhin daran gedacht, den Verformungskörper mit weiteren Sensoren an entsprechend gestalteten Biegeabschnitten auszustatten, so dass auch Quer- und Torsionskräfte messbar sind.

Die Erfindung betrifft ferner einen Verformungskörper gemäß Anspruch 15 für eine oben beschriebene Sensorvorrichtung mit einem einen Randabschnitt bildenden Stützabschnitt zum Abstützen des Verformungskörpers an einem Gehäuse und mit einem mit dem Stützabschnitt über mindestens einen Biegeabschnitt gekoppelten zentralen Krafteinleitungsabschnitt. Eine externe Kraft ist über eine mit dem Krafteinleitungsabschnitt koppelbare Druckplatte in den Krafteinleitungsabschnitt einleitbar. Der Krafteinleitungsabschnitt ist gegenüber dem Stützabschnitt über den Biegeabschnitt beweglich gelagert, und ein Sensor zur Messung einer Verformung des Verformungskörpers ist in dem Biegeabschnitt angeordnet. Insbesondere ist der Krafteinleitungsabschnitt über den Biegeabschnitt gegenüber dem Stützabschnitt verschwenkbar und/oder in der Krafteinleitungsrichtung versetzbar.

Mit einem solchen erfindungsgemäßen Verformungskörper in einer Sensorvorrichtung zur intraoralen Messung einer Kieferstellung, einer Kieferbewegung und/oder einer Kieferkraft sind besonders reproduzierbare und exakte Kieferrelationsbestimmungen möglich.

In der folgenden Beschreibung wird die Erfindung unter Bezugnahme auf die Zeichnung beispielhaft erläutert. In der Zeichnung zeigt:

Fig. 1    eine schematische Schnittansicht einer Sensorvorrichtung in einer parallel zu einer Krafteinleitungsrichtung (F) verlaufenden Schnittebene, die zur Erläuterung dargestellt ist,

Fig. 2    eine perspektivische Ansicht des Verformungskörpers der in Fig. 1 gezeigten Sensorvorrichtung,

Fig. 3    eine zweite perspektivische Ansicht des Verformungskörpers der in Fig. 1 gezeigten Sensorvorrichtung, bei dem die Biegeabschnitte bzw. die Biegestellen besonders markiert sind,

Fig. 4    eine schematische Darstellung des Verformungskörpers der in Fig. 1 gezeigten Sensorvorrichtung (Fig. 4a) sowie ein Koordinatensystem zur Erläuterung der Berechnung der Ortskoordinaten eines Krafteinteitungsortes aus den Messwerten der Sensoren (Fig. 4b),

Fig. 5    eine erste Ausführungsformen einer erfindungsgemäßen Sensorvorrichtung, bei der der Krafteinleitungsabschnitt (119) ein zentraler Abschnitt des Verformungskörpers und der Stützabschnitt (118) ein Randabschnitt des Verformungskörpers ist,

Fig. 6-8   verschiedene Ausführungsformen von Verformungskörpern für die in Fig. 5 gezeigte Ausführungsform der Erfindung.

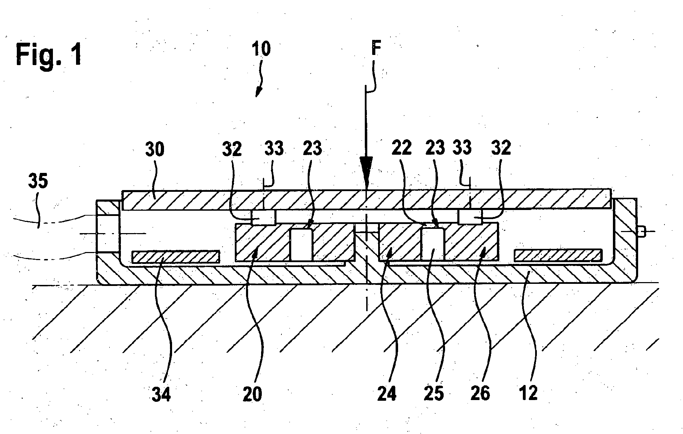

Fig. 1 ist eine schematische Schnittansicht einer Sensorvorrichtung 10 in einer parallel zu einer Krafteinleitungsrichtung F verlaufenden Schnittebene. Die Sensorvorrichtung besteht aus einem dosenförmigen Gehäuse 12 mit einem Boden und Seitenwänden, wobei das Gehäuse 12 oben durch eine flache Druckplatte 30 abgeschlossen ist. Ein Zwischenraum zwischen Gehäuse 12 und Druckplatte 30 ist abgedichtet, so dass keine Flüssigkeiten in das Gehäuseinnere eindringen können.

Das Gehäuse 12 und die Druckplatte 30 sind in einer senkrecht zur Krafteinleitungsrichtung F verlaufenden Schnittebene rund. Insgesamt ist die Sensorvorrichtung 10 etwa zylindenförmig mit einer parallel zur Krafteinleitungsrichtung F verlaufenden Zylinderachse. Die Sensorvorrichtung 10 kann vor Verwendung bei einer Kieferrelationsbestimmung in eine Halteplatte (nicht gezeigt) mit einer Haltevertiefung eingesetzt werden, wobei der Umriss der Halteplatte an die Abmessungen zwischen den beiden Zahnreihen des Unterkiefers eines Probanden angepasst ist. Die Halteplatte mit eingesetzter Sensorvorrichtung wird dann in den Bereich des Unterkiefers des Probanden eingesetzt und dort von den Zahnreihen festgehalten und fixiert. Ein Kraftinitiator (nicht gezeigt) mit einem Spitzenende zum Drücken auf die Druckplatte 30 der Sensorvorrichtung 10 in der Krafteinleitungsrichtung F wird in den Bereich des Oberkiefers des Probanden eingesetzt und dort festgehalten. Es werden dann sowohl die Kraft gemessen, mit der der Kraftinitiator auf die Druckplatte 30 drückt, als auch der Krafteinleitungsort, an dem der Kraftinitiator auf die Druckplatte 30 drückt. Dieser Krafteinleitungsort entspricht einer relativen Kieferstellung Wenn der Proband seinen Unterkiefer relativ zu seinem Oberkiefer bewegt, ändert sich der Krafteinleitungsort, so dass eine Bewegungsbahn des Kraftinitiators auf der Druckplatte (Kieferbewegung) registriert werden kann.

Im Inneren des Gehäuses unterhalb der Druckplatte 30 ist ein Verformungskörper 20 angeordnet. Der Verformungskörper 20 ist so mit der Druckplatte 30 verbunden, dass eine lokal auf die Druckplatte 30 einwirkende externe Kraft auf den Verformungskörper wirkt und diesen zumindest lokal in Biegeabschnitten 23 verformt. Die Verformung des Verformungskörpers 20 bei Krafteinwirkung wird mit Sensoren 22 erfasst, die an dem Verformungskörper in den Biegeabschnitten 23 angeordnet sind.

Der Verformungskörper 20 weist einen gegenüber dem Gehäuse 12 feststehenden Stützabschnitt 24 auf. Der Stützabschnitt 24 ist bei der ersten, in den Figuren 1-4 dargestellten Ausführungsform ein zentraler Abschnitt des Verformungskörpers 20, der in seiner Mitte auf einem Vorsprung des Gehäuses aufliegt und dort befestigt ist.

Der Stützabschnitt 24 ist über insgesamt drei Biegeabschnitte 23 mit einem Krafteinleitungsabschnitt 26 gekoppelt. Der Krafteinleitungsabschnitt umgibt den Stützabschnitt 24 etwa ringförmig. Wie in Fig. 1 gezeigt ist, ist die Druckplatte 30 unmittelbar an den Krafteinleitungsabschnitt 26, nicht jedoch an den Stützabschnitt 24 gekoppelt. Bei der dargestellten Ausführungsform ist die Druckplatte an drei Befestigungsstellen 33 mit dem Krafteinleitungsabschnitt 26 verschraubt. Andere Befestigungsarten sind gleichermaßen denkbar. Diese Befestigungsstellen weisen gegenseitig ebenso wie die Biegeabschnitte einen Winkel von 120° auf, so dass bei der dargestellten Ausführungsform eine Proportionalität zwischen den an den drei Befestigungsstellen einwirkenden Teillasten (-F1, -F2 und -F3) und den gemessenen Signalen gegeben ist. Bei Einwirkung einer Kraft auf die Druckplatte 30 verformt sich der Verformungskörper in den Biegeabschnitten 23 und der Krafteinleitungsabschnitt 26 wird gegenüber dem feststehenden Stützabschnitt 24 nach unten gedrückt (durch die in der Krafteinleitungsrichtung F wirkende Kraftkomponente) und gleichzeitig verkippt (sofern die Kraft nicht auf die Mitte der Druckplatte einwirkt).

Eine Stauchung und/oder eine Streckung einer Oberfläche des Verformungskörpers 20 in den Biegeabschnitten 23 infolge der Krafteinwirkung wird von den Sensoren erfasst und als Messsignal ausgegeben. Eine vektorielle Addition der drei Einzelsignale ergibt den Gesamtbetrag der in Axialrichtung F wirkenden Kraft. Die Sensoren können als Messwiderstände, insbesondere als Dehnungsmessstreifen (DMS) ausgebildet sein, deren Widerstand sich bei ihrer Streckung und Stauchung verändert.

Der Verformungskörper 20 ist einteilig etwa in Form einer flachen Kreisscheibe ausgebildet. Er wird vorzugsweise aus einem Metallstück wie etwa einer Metallplatte zusammenhängend und einteilig herausgeschnitten bzw. herausgefräst. Die Biegeabschnitte 23 werden hergestellt, indem die Kreisscheibe lokal geschwächt wird. Diese lokalen Schwächungen 25 können Durchbrüche durch die Kreisscheibe oder Materialabtragungen an der Metallplatte wie etwa Vertiefungen in der Platte sein. Bei den dargestellten Ausführungsformen weist der Verformungskörper jeweils mehrere Durchbrüche auf, zwischen denen sich die Biegeabschnitte 23 befinden.

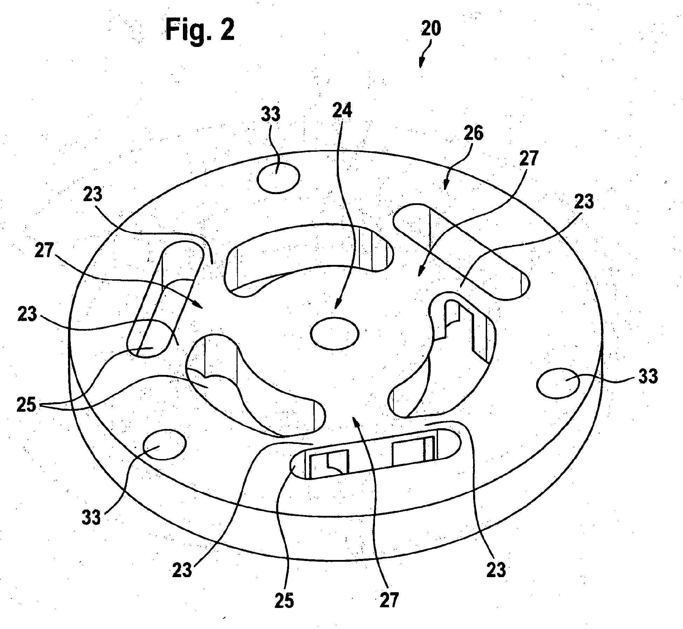

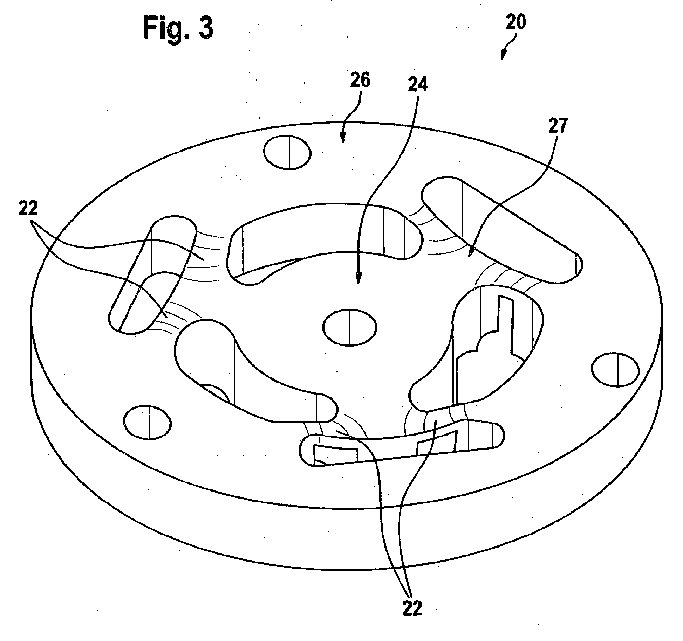

Wie besonders gut in den Figuren 2 und 3 gezeigt ist, weist der Verformungskörper 20 insgesamt drei als Doppelstege 27 ausgeführte Biegeabschnitte 23 auf. In jedem Biegeabschnitt ist ein Sensor 22 zur Messung einer Streckung und/oder Stauchung an einer Oberfläche des Stegs angeordnet. Jeder Doppelsteg 27 ist an einem zentralen Abschnitt davon einseitig mit dem Stützabschnitt 24 und an den beiden Stegenden in Längsrichtung des Stegs mit dem Krafteinleitungsabschnitt 26 verbunden. Die Längsachse der Doppelstege verläuft jeweils etwa senkrecht zu einer radial verlaufenden Richtung.

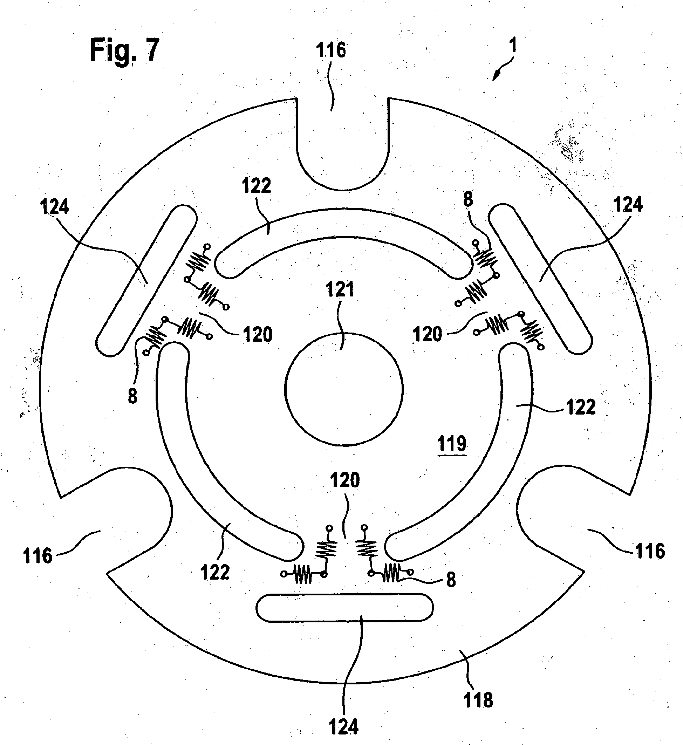

Eine mögliche Anordnung der Messwiderstände an den Doppelstegen ist in Fig. 7 gezeigt. Jeder Doppelsteg trägt vier Dehnungsmessstreifen, die zu einer Vollbrücke geschaltet sind und einen Sensor bilden.

Anstelle der Doppelstege können einfache, in Radialrichtung verlaufende Stege vorgesehen sein, wie sie etwa in Fig. 6 gezeigt sind. Die in Radialrichtung verlaufenden Stege tragen jeweils mindestens einen Messwiderstand eines Sensors, wobei der Messwiderstand ausgehend von einem äußeren Rand des Stützabschnitts an der Oberfläche des Stegs radial nach außen in Richtung auf den Krafteinleitungsabschnitt verläuft. Besonders bevorzugt weist jeder Steg vier Messwiderstände auf, die jeweils gemäß der in Fig. 6 gezeigten Anordnung auf die Stege aufgebracht sind. Dabei sind zwei Messwiderstände an einer radial inneren Oberfläche des Stegs und zwei weitere Messwiderstände an einer radial äußeren Oberfläche des Stegs derart angeordnet, dass bei einer Krafteinleitung in den Krafteinleitungsabschnitt die radial innere Oberfläche des Stegs in dem Maß gestreckt wird, in dem die radial äußere Oberfläche gestaucht wird und umgekehrt (bei Krafteinleitung wird der Steg etwa S-förmig verformt).

Wenn ein Sensor vier zu einer Vollbrücke geschaltete Dehnungsmessstreifen hat, wird die Temperaturabhängigkeit reduziert und die Brückenspannung gegenüber einer Halbbrücke verdoppelt. Dadurch wird die Messgenauigkeit erhöht.

Die vier Dehnungsmessstreifen eines Sensors bzw. die 12 Dehnungsmessstreifen von insgesamt drei Sensoren oder auch weitere Dehnungsmessstreifen können in einem einzigen Beschichtungsschritt auf der Oberfläche des Verformungskörpers aufgebracht werden.

Insgesamt hat der Verformungskörper 20 eine 3 zählige Radiärsymmetrie. Benachbarte Biegeabschnitte weisen gegenseitig entsprechend einen Winkel von 120°auf.

Zwischen dem Verformungskörper 20 und der Druckplatte 30 sind Abstandhalteelemente 32 angeordnet, so dass der Krafteinleitungsabschnitt 26 auf Distanz zu der Druckplatte 30 gehalten wird. Der Krafteinleitungsabschnitt hat dann einen erforderlichen Freiraum für eine Bewegbarkeit gegenüber dem Stützabschnitt. Eine "Bewegbarkeit" im Sinne der vorliegenden Erfindung ist bereits dann gegeben, wenn die Oberflächen der Biegeabschnitte bei Krafteinleitung geringfügig steckbar und/oder stauchbar sind. Entsprechend genügen Abstandhalteelemente mit einer Abmessung in Krafteinleitungsrichtung (F) von weniger als 1 mm ohne Weiteres.

Fig. 1 zeigt besonders deutlich, dass der Durchmesser der Druckplatte 30 fast doppelt so groß ist wie der Durchmesser des Verformungskörpers 20. Der Freiraum unterhalb der Druckplatte 30 seitlich des Verformungskörpers kann zum Einbau einer Platine 34 mit einer Verstärkerschaltung zur Verstärkung des Messsignals oder einer Auswertschaltung zur Auswertung des Messsignals verwendet werden. Das Gehäuse 12 weist seitlich einen Kabelausgang 35 zum Herausführen eines Messsignals aus der Sensorvorrichtung auf.

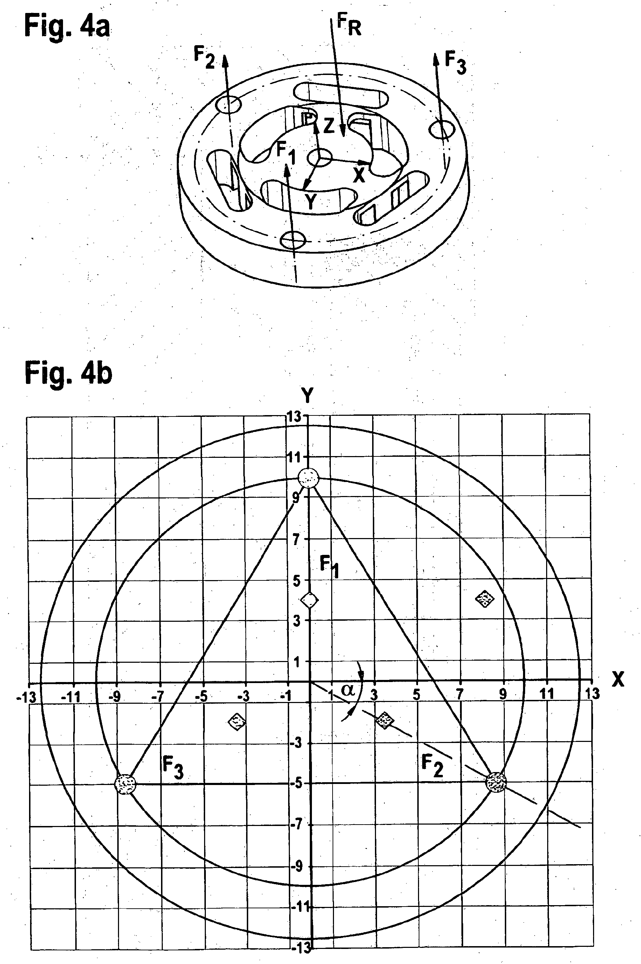

Anhand Fig. 4 wird im Folgenden erläutert, wie die Örtskoordinaten eines Krafteinleitungsortes auf der Druckplatte aus den Messwerten der Sensoren bestimmbar sind.

$F_R$ in Fig. 4a entspricht der eingeleiteten Axialkraft. $F_1$, $F_2$ und $F_3$ sind die an den drei Befestigungsstellen 33 wirkenden Gegenkräfte des Verformungskörpers. Die Position des Krafteinleitungsortes wird in kartesischen Koodinaten $x_s$, $y_s$ mit dem Mittelpunkt der Druckplatte als Koordinatenursprung ausgedrückt. Es wird ein Kräftegleichgewicht unter den folgenden Randbedingungen gebildet:

$$(1) \qquad \sum_i F_{iz} = 0$$

$$(2) \qquad \sum_i M_{ix} = 0$$

$$(3) \qquad \sum_i M_{iy} = 0 \; ;$$

wobei $M_{ix}$ und $M_{iy}$ die Momente in der x-Richtung und in der y-Richtung sind.

Über die Momentengleichgewichte lassen sich $x_s$ und $y_s$ wie folgt bestimmen:

$$(4) \qquad x_s = \frac{F_1 \cdot 0 + F_2 \cdot \cos(\alpha) \cdot r - F_3 \cdot \cos(\alpha) \cdot r}{F_1 + F_2 + F_3}$$

$$(5) \qquad y_{s=} \frac{F_1 \cdot r - F_2 \cdot \sin(\alpha) \cdot r - F_3 \cdot \sin(\alpha) \cdot r}{F_1 + F_2 + F_3}$$

Wie man in Fig. 4b erkennen kann, ist bei der Anordnung der Sensoren unter einem Winkel von jeweils 120° gemäß der dargestellten Aus führungsform und der dargestellten Lage des Koordinatensystems der Winkel $\alpha$ gleich 30°.

Der Abstand r und der Winkel $\alpha$ sind konstant. Da sich die Teilkräfte proportional zu den gemessenen Messwerten $\frac{mV}{V}$ verhalten, lässt sich die Gleichung zur Ortsbestimmung auch direkt mit den drei gemessenen Messwerten $U_1$, $U_2$ und $U_3$ verwenden, ohne zuvor die Kräfte zu bestimmen.

Wie der Fachmann leicht erkennen wird, können die drei Biegeabschnitte auch unter anderen, ggf. auch unterschiedlichen gegenseitigen Winkeln und Abständen vom Koordinatenursprung angeordnet sein. Die Formeln (4) und (5) sind dann entsprechend anzupassen, wobei dann ggf. drei Winkel a, $\beta$ und $\gamma$ und drei Abstände $r_1$, $r_2$ und $r_3$ zu verwenden sind.

Aus den drei Messwerten lassen sich auf diese Weise die Koordinaten des Krafteinleitungsortes auf der Druckplatte bestimmen und diese können dann an einer Anzeigevorrichtung angezeigt werden.

Eine erste Ausführungsform der Erfindung ist in Fig. 5 dargestellt. Bei dieser Ausführungsform ist der Krafteinleitungsabschnitt 119 ein zentraler Abschnitt des Verformungskörpers und der Stützabschnitt 118 ist ein den zentralen Abschnitt ringartig umlaufender Randabschnitt des Verformungskörpers.

Fig. 5 zeigt die Ausführungsform in einer Schnittansicht. Die dargestellte Sensorvorrichtung hat ein dosenförmiges Gehäuse 3, das in seinem Inneren einen Verformungskörper 1 aufnimmt, und das oben von einer Druckplatte 4 abgeschlossen ist. Am Innenumfang eines Wandabschnitts des dosenförmigen Gehäuses 3 ist eine umlaufende Nut 132 ausgebildet, in die ein Ring 45 einsetzbar ist, der in eine Umfangsnut 46 der Druckplatte 4 eingreift. Sowohl die Nut 132 als auch die Umfangsnut 46 sind mit hinreichendem Spiel versehen, so dass sich die Druckplatte 4 in Fig. 1 in abwärtiger Richtung bewegen kann. Der eingesetzte Ring 45 dient dabei als Anschlagelement, das eine Zerstörung des Verformungskörpers, der mit der Druckplatte 4 gekoppelt ist, verhindert. Die Druckplatte 4 hat eine oberhalb der Umfangsnut 46 ausgebildete Umfangsnut 47, in der eine Dichtung 43 aufgenommen ist. Die Dichtung 43 ist aus einem Elastomer gefertigt und hat eine in Umfangsrichtung vorstehende Dichtlippe 44, die mit der Wandung des dosenförmigen Gehäuses 3 in dichtender Anlage ist. Die Dichtung 43 ist ausgelegt, eine hinreichende Bewegung der Druckplatte 4 relativ zu dem Gehäuse 3 zuzulassen. Alternativ kann die Dichtung auch als Balg ausgeführt sein, der an seinen beiden Umfangskanten fest mit dem jeweiligen Element (Gehäuse/Druckplatte) verbunden ist.

Die Druckplatte 4 ist eine Kreisscheibe mit einem zentralen Vorsprung 42, der seinerseits einen Ringbund 46 hat, der mit dem Verformungskörper 1 in Anlage bringbar ist.

Im Inneren des dosenförmigen Gehäuses 3, d.h. im Bereich des Gehäusebodens ist eine Bodenfläche 133 mit einer Aussparung 131 angeordnet. Die Aussparung 131 ist angepasst, eine Auswertplatine 5 aufzunehmen, auf der nicht näher beschriebene Elektronikbauteile und Leitungen angebracht sind, die angepasst sind, Widerstandswerte von Messwiderständen zu erfassen, auszuwerten und das Ergebnis nach draußen weiterzuleiten.

Oberhalb der Aussparung 131 ist der Verformungskörper 1 angeordnet. Der Verformungskörper 1 hat einen zentralen Krafteinleitungsabschnitt 119 und einen Stützabschnitt 118, der über Biegeabschnitte mit dem Krafteinleitungsabschnitt 119 verbunden ist.

Im Umfangsbereich des Verformungskörpers 1 ist eine Randverstärkung 117 ausgebildet, mit der der Verformungskörper 1 im eingebauten Zustand auf der Bodenfläche 133 des Gehäuses 3 aufliegt. Verformungskörper 1 und Randverstärkung 117 sind im Stützabschnitt 118 durchbohrt und Schrauben 6, die in das Gehäuse 3 eingeschraubt werden, legen den Verformungskörper an der Bodenfläche des Gehäuses 3 fest. Alternativ dazu kann, wie auf der linken Seite in Fig. 5 gezeigt ist, ein Zwischenring 66 verwendet werden, der von der Schraube 6 durchdrungen wird. Dieser Zwischenring 66 verteilt einerseits die Befestigungskräfte auf einen größeren Bereich des Stützabschnitts 118 des Verformungskörpers 1 und ermöglicht zudem eine Gestaltung des Verformungskörpers mit randseitig offenen Kerben zur Befestigung. Durch großzügig bemessene Kerben ist es möglich, ungewollte Verspannungen in dem Verformungskörper 1 durch das Festschrauben zu verhindern.

In der in Fig. 5 gezeigten Anordnung sind die Messwiderstände (nicht gezeigt) auf der oberen Seite des Verformungskörpers 1 in den Biegeabschnitten angeordnet. In diesem Fall können die als Durchbrüche 122 gezeigten Schwächungen des Verformungskörpers für die Leitungsführung 51 zwischen den Messwiderständen und der Auswertelektronik 5 herangezogen werden.

Die Figuren 6 bis 8 zeigen verschiedene Ausführungsformen von Verformungskörpern für die in Fig. 5 gezeigte Ausführungsform der Erfindung. Diese Verformungskörper weisen jeweils einen zentralen Krafteinleitungsabschnitt 119 und einen den Krafteinleitungsabschnitt 119 ringartig umlaufenden Stützabschnitt 118 auf.

Die Stützabschnitte 118 der in den Figuren 6 und 7 gezeigten Ausführungsformen weisen jeweils an ihrem Umfang Kerben 116 auf, durch die Befestigungselemente führbar sind, um den Verformungskörper 1 an dem Gehäuse 3 zu befestigen. Der Krafteinleitungsabschnitt 119 hat jeweils in seiner Mitte ein Loch 121, das so bemessen ist, dass ein Vorsprung 42 der Druckplatte 4 angreifen kann.

Der Krafteinleitungsabschnitt 119 des in Fig. 6 gezeigten Verformungskörpers ist über drei radial nach außen verlaufende. Stege 120 mit dem Stützabschnitt 118 verbunden, die jeweils zueinander einen Winkel von 120° einschließen. Jeder Steg 120 trägt an seiner Oberfläche einen Sensor 8, wobei jeder Sensor insgesamt vier Messwiderstände aufweist, die zu einer Vollbrücke geschaltet sind. Bei einer nicht-zentralen Krafteinwirkung auf die Druckplatte 4 werden die Oberflächen der drei Stege 120 unterschiedlich stark verformt, wobei diese Verformungen gemessen werden. Aus den drei Messwerten kann der Krafteinleitungsort auf der Druckplatte und/oder der Kraftbetrag bestimmt werden (siehe oben).

Dabei können jeweils zwei Messwiderstände eines Sensors 8 in einem Streckabschnitt und zwei Messwiderstände des Sensors 8 in einem Stauchabschnitt angeordnet sein (siehe oben), um Störgrößen wie etwa Temperatur zufriedenstellend kompensieren zu können.

Ein alternativer Verformungskörper, der Doppelstege anstelle der einfachen Stege aufweist, ist in Fig. 7 dargestellt. Die Doppelstege entstehen durch eine geeignete Anordnung von Gruppen von Durchbrüchen 122, 124 durch den Verformungskörper.

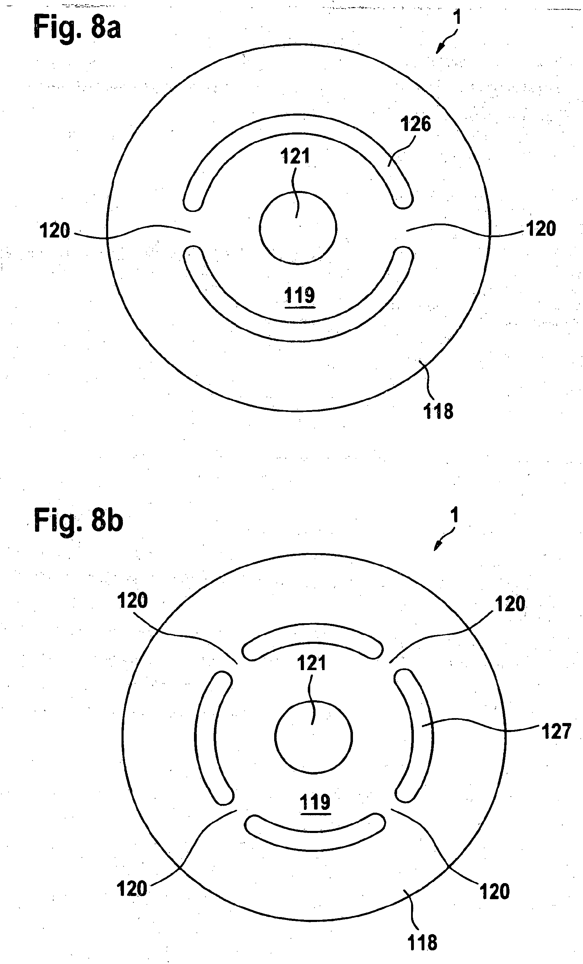

Weitere alternative Ausführungsformen erfindungsgemäßer Verformungskörper mit zwei bzw. vier Biegeabschnitten 120 und Gruppen von Durchbrüchen 126 bzw. 127 sind in den Figuren 8a und 8b gezeigt.

Eine erfindungsgemäße Sensorvorrichtung ist nicht auf die beschriebene Ausführungsform beschränkt. Vielmehr ist es für den Fachmann offensichtlich, dass der Verformungskörper anders geformt sein kann und mehr oder weniger Biegeabschnitte und Sensoren aufweisen kann als bei der beschriebenen Ausführungsform. So genügt bereits eine Biegestelle mit einem einzigen Sensor zur Messung des Betrags einer Kieferkraft. Andererseits können mehr als drei Sensoren vorgesehen sein, wenn auch eine Krafteinleitungsrichtung, eine Torsion, eine Querkraft o. dgl. gemessen werden soll. Anstelle der Stege können anders geformte Biegeabschnitte vorhanden sein. Bspw. können die Biegestellen in Form von Schwächungen des Verformungskörpers gebildet sein, ohne dass notwendigerweise Stege vorhanden sind. Pro Biegeabschnitt kann auch nur ein Sensor mit einem einzigen Dehnungsmessstreifen vorhanden sein, der in einer Viertelbrücke geschaltet ist. Auch Halbbrücken mit jeweils zwei Dehnungsmessstreifen sind vorstellbar. Die einzelnen Dehnungsmessstreifen einer Brücke können an verschiedenen Oberflächeabschnitten, in unterschiedlichen Ausrichtungen und geometrischen Anordnungen an der Oberfläche des Verformungskörpers angeordnet sein. Verschiedene Arten von Brückenschaltungen zur Kompensation verschiedener Störgrößen sind aus dem Stand der Technik bekannt.

## Patentansprüche

1. Sensorvorrichtung (10) zur intraoralen Messung einer Kieferstellung und/oder einer Kieferbewegung mit
einem Gehäuse (12),
einem in dem Gehäuse (12) aufgenommenen Verformungskörper (20),
einer mit dem Verformungskörper (20) gekoppelten Druckplatte (30), in die lokal eine externe Kraft (F) einleitbar ist, so dass sich der Verformungskörper (20) zumindest lokal verformt,
wobei der Verformungskörper (20) folgendes aufweist: einen gegenüber dem Gehäuse (12) feststehenden Stützabschnitt (24), einen Krafteinleitungsabschnitt (26), auf den die in die Druckplatte einzuleitende externe Kraft (F) einwirkt, und mindestens zwei, bevorzugt drei den Stützabschnitt (24) mit dem Krafteinleitungsabschnitt (26) verbindende Biegeabschnitte (23), die bei Einleitung der externen Kraft (F) in den Krafteinleitungsabschnitt (26) verformbar sind und an denen jeweils ein Sensor (22) zur Messung der Verformung des Verformungskörpers angeordnet ist, **dadurch gekennzeichnet, dass**
der Krafteinleitungsabschnitt (26) ein zentraler Abschnitt des Verformungskörpers und der Stützabschnitt (24) ein den zentralen Abschnitt zumindest teilweise umlaufender Randabschnitt des Verformungskörpers ist.

2. Sensorvorrichtung (10) nach Anspruch 1, **dadurch gekennzeichnet, dass** der Verformungsköper (20) eine einteilig gebildete Platte, bevorzugt eine einteilige Metallplatte ist.

3. Sensorvorrichtung nach Anspruch 1 oder 2, **dadurch gekennzeichnet, dass** der Verformungskörper (20) in der Umgebung des Biegeabschnitts (23) mindestens eine lokale Schwächung (25) aufweist.

4. Sensorvorrichtung nach Anspruch 3, **dadurch gekennzeichnet, dass** die lokale Schwächung (25) in Form eines oder mehrerer Durchbrüche durch den Verformungskörper (20), einer Materialabtragung an dem Verformungskörper (20) und/oder einer lokalen Verringerung der Stärke des Verformungskörpers (20) gebildet ist.

**5.** Sensorvorrichtung nach Anspruch 3 oder 4, **dadurch gekennzeichnet, dass** der Biegeabschnitt (23) in Form eines den Krafteinleitungsabschnitt (26) mit dem Stützabschnitt (24) verbindenden Stegs (27), bevorzugt eines Doppelstegs, gebildet ist.

**6.** Sensorvorrichtung nach Anspruch 5, **dadurch gekennzeichnet, dass** der Sensor (22) an dem und/oder angrenzend an den Steg angeordnet ist.

**7.** Sensorvorrichtung nach einem der vorhergehenden Ansprüche, **dadurch gekennzeichnet, dass** die Biegeabschnitte (23) jeweils als sich quer, insbesondere etwa senkrecht zur Radialrichtung erstreckende Doppelstege ausgeführt sind, die jeweils in ihrer Mitte mit dem zentralen Abschnitt des Verformungskörpers und an ihren beiden Endabschnitten mit dem Randabschnitt verbunden sind oder umgekehrt.

**8.** Sensorvorrichtung nach einem der vorhergehenden Ansprüche, **dadurch gekennzeichnet, dass** der Verformungskörper im Wesentlichen eine 3-zählige Radiärsymmetrie hat.

**9.** Sensorvorrichtung nach einem der vorhergehenden Ansprüche, **dadurch gekennzeichnet, dass** der Sensor (22) mindestens einen Messwiderstand, bevorzugt einen Dehnungsmessstreifen aufweist.

**10.** Sensorvorrichtung nach Anspruch 9, **dadurch gekennzeichnet, dass** der Sensor vier zu einer Vollbrücke geschaltete Dehnungsmessstreifen aufweist.

**11.** Sensorvorrichtung nach Anspruch 9 oder 10, **dadurch gekennzeichnet, dass** der Sensor mindestens einen entlang einer ersten Biegefläche des Biegeabschnitts angeordneten ersten Dehnungsmessstreifen und einen entlang einer zweiten Biegefläche des Biegeabschnitts angeordneten zweiten Dehnungsmessstreifen aufweist, wobei die erste Biegefläche infolge einer Krafteinwirkung auf die Druckplatte gestreckt wird, wenn die zweite Biegefläche gestaucht wird und umgekehrt, und wobei der erste Dehnungsmessstreifen und der zweite Dehnungsmessstreifen zu einer Halbbrücke oder einer Vollbrücke geschaltet sind.

**12.** Sensorvorrichtung nach einem der vorhergehenden Ansprüche, **dadurch gekennzeichnet, dass** der Krafteinleitungsabschnitt (26) fest mit der Druckplatte (30) gekoppelt ist, so dass die relative Läge von Druckplatte und Krafteinleitungsabschnitt bei Krafteinwirkung auf die Druckplatte unveränderlich ist.

**13.** Sensorvorrichtung nach einem der vorhergehenden Ansprüche, **dadurch gekennzeichnet, dass** das Gehäuse (10) in einer senkrecht zur Krafteinleitungsrichtung verlaufenden Schnittebene abgerundet, bevorzugt kreisförmig ist, und derart geformt ist, dass es zwischen die Molaren eines Unterkiefers einsetzbar ist.

**14.** Sensorvorrichtung nach einem der vorhergehenden Ansprüche, **dadurch gekennzeichnet, dass** eine Abmessung einer Krafteinleitungsfläche der Druckplatte (30) größer ist, bevorzugt mehr als 1,5 mal so groß ist, insbesondere etwa doppelt so groß ist oder größer als eine maximale Abmessung des Verformungskörpers (20) in einer senkrecht zur Krafteinleitungsrichtung (F) verlaufenden Richtung.

**Claims**

**1.** A sensor device (10) for the intraoral measurement of a jaw position and/or a jaw movement, comprising
a housing (12),
a deformation body (20) accommodated within the housing (12),
a pressure plate (30) coupled to the deformation body (20) and into which an external force (F) can be introduced locally so that the deformation body (20) deforms, at least locally,
the deformation body (20) comprising the following: a support portion (24) that is fixed with respect to the housing (12), a force introduction portion (26) on which the external force (F) to be introduced into the pressure plate acts, and at least two, preferably three, bending portions (23) connecting the support portion (24) to the force introduction portion (26) which can be deformed when the external force (F) is introduced into the force introduction portion (26) and on each of which a sensor (22) for measuring the deformation of the deformation body is disposed, **characterised in that**
the force introduction portion (26) is a central portion of the deformation body and the support portion (24) is an edge portion of the deformation body at least partially running round the central portion.

2. The sensor device (10) according to Claim 1, **characterised in that** the deformation body (20) is a plate formed in one part, preferably a one-part metal plate.

3. The sensor device according to Claim 1 or 2, **characterised in that** the deformation body (20) has at least one local weakening (25) in the proximity of the bending portion (23).

4. The sensor device according to Claim 3, **characterised in that** the local weakening (25) is made in the form of one or more breaks through the deformation body (20), removal of material on the deformation body (20) and/or local reduction of the thickness of the deformation body (20).

5. The sensor device according to Claim 3 or 4, **characterised in that** the bending portion (23) is made in the form of a bar (27), preferably a double bar, connecting the force introduction portion (26) to the support portion (24).

6. The sensor device according to Claim 5, **characterised in that** the sensor (22) is disposed on and/or adjacent to the bar.

7. The sensor device according to any of the preceding claims, **characterised in that** the bending portions (23) are each made as double bars extending transversely, in particular approximately perpendicularly to the radial direction, which are each connected in their middle to the central portion of the deformation body and at their two end portions to the edge portion or vice versa.

8. The sensor device according to any of the preceding claims, **characterised in that** the deformation body has substantially three-fold radial symmetry.

9. The sensor device according to any of the preceding claims, **characterised in that** the sensor (22) has at least one measuring resistor, preferably a strain gauge.

10. The sensor device according to Claim 9, **characterised in that** the sensor has four strain gauges connected to form a full bridge.

11. The sensor device according to Claim 9 or 10, **characterised in that** the sensor has at least one first strain gauge disposed along a first bending surface of the bending portion and a second strain gauge disposed along a second bending surface of the bending portion, the first bending surface being stretched onto the pressure plate as the result of the effect of force when the second bending surface is compressed, and vice versa, and the first strain gauge and the second strain gauge being connected to form a half bridge or a full bridge.

12. The sensor device according to any of the preceding claims, **characterised in that** the force introduction portion (26) is coupled securely to the pressure plate (30) so that the relative position of the pressure plate and the force introduction portion is unchanged when force acts upon the pressure plate.

13. The sensor device according to any of the preceding claims, **characterised in that** the housing (10) is rounded, preferably circular, in a section plane running perpendicular to the direction of introducing force, and is formed such that it can be inserted between the molars of a lower jaw.

14. The sensor device according to any of the preceding claims, **characterised in that** a dimension of a force introduction surface of the pressure plate (30) is greater, preferably more than 1.5 times as great, in particular approximately twice as great or greater than a maximum dimension of the deformation body (20) in a direction running perpendicular to the direction of introducing force (F).

## Revendications

1. Dispositif de détection (10) pour la mesure intra-orale de la position et/ou du déplacement de la mâchoire, comportant un boîtier (12),
   un élément déformable (20) logeant dans le boîtier (12),
   une plaque de pression (30) couplée à l'élément déformable (20) et dans laquelle une force externe (F) peut être localement induite de manière à déformer au moins localement l'élément déformable (20),
   dans lequel l'élément déformable (20) présente les éléments suivants : un segment d'appui (24) fixe par rapport au boîtier (12), un segment d'induction de force (26) sur lequel intervient la force externe (F) destinée à être induite dans la plaque de pression, et au moins deux et de préférence trois segments de flexion (23) qui relient le segment d'appui (24) au segment d'induction de force (26) et sont déformables sous l'effet de l'induction de la force externe (F) dans le segment d'induction de force (26) et sur lesquels un capteur (22) est respectivement agencé pour mesurer la déformation de l'élément déformable, **caractérisé en ce que** le segment d'induction de force (26) consiste en une partie centrale de l'élément déformable, et le segment d'appui (24) consiste en un segment de bord de l'élément déformable qui entoure au moins partiellement ladite partie centrale.

2. Dispositif de détection (10) selon la revendication 1, **caractérisé en ce que** l'élément déformable (20) consiste en une plaque unitaire, de préférence en une plaque métallique unitaire.

3. Dispositif de détection selon la revendication 1 ou 2, **caractérisé en ce que** l'élément déformable (20) présente au moins une fragilisation locale (25) à proximité du segment de flexion (23).

4. Dispositif de détection selon la revendication 3, **caractérisé en ce que** la fragilisation locale (25) est constituée sous la forme d'une ou de plusieurs perforations dans l'élément déformable (20), d'une ablation de matière au niveau de l'élément déformable (20) et/ou d'une diminution locale de l'épaisseur de l'élément déformable (20).

5. Dispositif de détection selon la revendication 3 ou 4, **caractérisé en ce que** le segment de flexion (23) est constitué sous la forme d'une passerelle (27) reliant le segment d'induction de force (26) au segment d'appui (24), de préférence sous la forme d'une passerelle double.

6. Dispositif de détection selon la revendication 5, **caractérisé en ce que** le capteur (22) est disposé sur la passerelle et/ou adjacent à celle-ci.

7. Dispositif de détection selon l'une des revendications précédentes, **caractérisé en ce que** les segments de flexion (23) sont chacun conçus sous la forme de passerelles doubles qui sont dirigées de manière transversale, notamment de manière approximativement perpendiculaire au sens radial, et qui sont chacune reliées par leur milieu à la partie centrale de l'élément déformable et par leurs deux extrémités terminales au segment de bord, ou inversement.

8. Dispositif de détection selon l'une des revendications précédentes, **caractérisé en ce que** l'élément déformable présente essentiellement une symétrie radiée d'ordre 3.

9. Dispositif de détection selon l'une des revendications précédentes, **caractérisé en ce que** le capteur (22) présente au moins une résistance de mesure, de préférence une jauge de contrainte.

10. Dispositif de détection selon la revendication 9, **caractérisé en ce que** le capteur présente quatre jauges de contrainte montées pour former un pont complet.

11. Dispositif de détection selon la revendication 9 ou 10, **caractérisé en ce que** le capteur présente au moins une première jauge de contrainte disposée le long d'une première surface de flexion du segment de flexion et une deuxième jauge de contrainte disposée le long d'une deuxième surface de flexion du segment de flexion, la première surface de flexion subissant un étirement à la suite de l'intervention d'une force sur la plaque de pression, lorsque la deuxième surface de flexion est soumise à un écrasement, et inversement, la première jauge de contrainte et la deuxième jauge de contrainte étant montées pour former un demi-pont ou un pont complet.

12. Dispositif de détection selon l'une des revendications précédentes, **caractérisé en ce que** le segment d'induction de force (26) est couplé fixe à la plaque de pression (30), si bien que la position relative de la plaque de pression et du segment d'induction de force demeurent inchangées lors de l'intervention d'une force sur la plaque de pression.

13. Dispositif de détection selon l'une des revendications précédentes, **caractérisé en ce que** le boîtier (10) est arrondi dans un plan sécant dirigé perpendiculairement au sens d'induction de force, en étant de préférence circulaire, et est façonné de manière à pouvoir être inséré entre les molaires de la mâchoire inférieure.

14. Dispositif de détection selon l'une des revendications précédentes, **caractérisé en ce qu'**une dimension d'une surface d'induction de force de la plaque de pression (30) est supérieure à une dimension maximale de l'élément déformable (20) dans un sens dirigé perpendiculairement au sens d'induction de force (F), en étant de préférence plus d'1,5 fois plus grande, notamment environ deux fois plus grande, voire davantage.

13

## Fig. 1

# Fig. 2

20

33    24    26    27

23

27

23

23

25

33

23    25    27    23

33

**Fig. 3**

20

26 24 27

22

22

## Fig. 4a

## Fig. 4b

Fig. 5

**Fig. 6a**

**Fig. 6b**

## Fig. 7

**Fig. 8a**

1

126

121

120

120

119

118

**Fig. 8b**

1

120

120

121

127

119

120

120

118

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005006323 A1

- WO 9203106 A

**In der Beschreibung aufgeführte Nicht-Patentliteratur**

- **A. DIETZEL ; A. ZÖLLNER.** Zur Lagebestimmung des Unterkiefers in zentrischer Relation mit dem DIR-System. *Deutscher Ärzte Verlag, DZZ, Deutsche Zahnärztliche Zeitschrift,* 2012, vol. 67 (2